# EUROPÄISCHE PATENTSCHRIFT

(11) **EP 1 466 638 B1**
(45) Veröffentlichungstag und Bekanntmachung des Hinweises auf die Patenterteilung: **11.07.2007**
(21) Anmeldenummer: 03028771.8
(22) Anmeldetag: 13.12.2003
(51) Int. Cl.: A61M 5/28, A61M 5/32, A61M 5/00, A61M 5/50, A61M 5/34

(54) **Vorgefüllte Spritze oder Karpule für medizinische Zwecke**
Prefilled syringe or carpule for medical purposes
Seringue préremplie ou carpule à usage médical

(30) Priorität: 09.04.2003 DE 10316127
(43) Veröffentlichungstag der Anmeldung: 13.10.2004
(73) Patentinhaber: Arzneimittel GmbH Apotheker Vetter & Co. Ravensburg, 88212 Ravensburg (DE)
(72) Erfinder: Vetter, Udo, J., 88214 Ravensburg (DE); Schütz, Andreas, Dr., 82152 Krailling (DE); Glocker, Joachim, 88250 Weingarten (DE)
(74) Vertreter: Dziewior, Joachim

(56) Entgegenhaltungen:
- EP-A- 0 528 120
- CH-A- 75 287
- DE-A1- 19 638 940
- DE-A1- 19 751 219
- US-A- 6 053 892

## Beschreibung

Die Erfindung betrifft eine vorgefüllte Spritze oder Karpule für medizinische Zwecke, mit einem Spritzenzylinder aus Glas und einem darin angeordneten, mittels einer Kolbenstange verschiebbaren Spritzenkolben. Weiter betrifft die Erfindung ein Verfahren zur Konfektionierung einer derartigen Spritze.

Spritzen der eingangs genannten Art sind in vielfältigen Ausführungsformen bekannt, wobei deren Gestaltung im einzelnen häufig von der Prozessführung bei der Reinigung, Befüllung und Konfektionierung abhängt. Ein grundsätzliches Problem hierbei stellen insbesondere auch Hochtemperatur-Prozesse dar, insbesondere dann, wenn die Fertigspritze bereits mit fest eingesetzter Kanüle ausgestattet sein soll.

Aus dem Stand der Technik ist beispielsweise die Patentschrift EP-A-0 528 120 bekannt. In dieser Schrift wird eine kanülenlose Fertigspritze offenbart, bei welcher in einer ersten Raststellung einer Sicherungskappe die Lyophilisierung (Vakuumgefriertrocknung) eines in gelöster Form in den Spritzenzylinder abgefüllten Wirkstoffs erfolgt, wobei ein Lösungsmittel über einen Ringspalt, einen Ringraum und Ausnehmungen in einem Anschlussteil entweichen kann. Die Spritze wird dann unmittelbar im Anschluss an die Lyophilisierung in steriler Umgebung durch Überführen der Sicherungskappe in eine zweite Raststellung geschlossen und mittels eines Sicherungsrings gesichert. Der Sicherungsring ist über einen als Sollbruchstelle ausgebildeten Anschlusssteg an die Sicherungskappe angeschlossen.

Der Erfindung liegt die Aufgabe zugrunde, eine Spritze der eingangs genannten Art zu schaffen, die erweiterte Möglichkeiten in der Prozessführung eröffnet, gleichwohl auf einfache und damit kostengünstige Weise konfektioniert werden kann.

Eine diese Aufgabe lösende Spritze oder Karpule ist versehen mit einer am kanülenseitigen Ende des Spritzenzylinders axial aufgesetzten, im Rastsitz gehaltenen Kanülenkappe, in die eine Kanüle fest eingesetzt ist, ferner mit einer zur Kanülenkappe koaxial angeordneten, in die Kanülenkappe eingesetzten Verschlußscheibe, die einerseits stirnseitig dem Spritzenzylinder und andererseits der innenseitigen Stirnfläche der Kanülenkappe anliegt und mittig eine Ausnehmung in Form eines Sacklochs aufweist, deren Boden eine Membran bildet, wobei das dem Spritzenzylinder zugewandte Ende der Kanüle über die innenseitige Stirnfläche der Kanülenkappe frei in die Ausnehmung der Verschlußscheibe vorsteht, sowie mit einem aus einer Verschlußkappe und einem daran über eine Sollbruchstelle angeschlossenen Klemmring bestehenden Verschlußteil, das von einer ersten, an der Außenmantelfläche der Kanülenkappe für den Klemmring ausgebildeten Rastposition, die eine Sterilisierung des Verschlußteilinnenraums ermöglicht, in eine zweite, die Kanülenkappe steril abdichtend umschließende Rastposition verstellbar ist.

Der durch die Erfindung erreichte Vorteil besteht im wesentlichen darin, dass der aus Glas bestehende Spritzenzylinder vor der Befüllung ohne weiteres Hochtemperaturprozessen unterworfen werden kann, insbesondere also einer Einbrennsilikonisierung unterzogen werden kann, ohne dass hierdurch Probleme auftreten, wie dies bei in den Glaskörper eingeklebten Kanülen der Fall ist. Darüber hinaus ist die Kanüle im Lagerzustand sowohl gegen äußere Einflüsse als auch gegen Kontakt mit der Arzneistofflösung über die Verschlußscheibe geschützt. Zusätzlich besteht die Möglichkeit, den Spritzenzylinder bzw. die Karpule blasenfrei zu befüllen, was die Handhabung vor der Anwendung vereinfacht. Darüber hinaus findet bei der Anwendung eine Selbstaktivierung statt, da die in der Verschlußscheibe gebildete Membran von der ihr zugewandten Spitze der Kanüle unter dem von der Flüssigkeit weitergeleiteten Druck der Kolbenstange durchstochen wird.

Um einen zusätzlichen Schutz insbesondere der Kanüle zu erreichen, ist in vorteilhafter Weiterbildung der Erfindung vorgesehen, dass im Inneren des Verschlussteils eine Nadelschutzkappe angeordnet ist. Diese Nadelschutzkappe besteht zweckmäßigerweise aus Gummi.

Desweiteren wird im Rahmen der Erfindung vorgeschlagen, dass die Kanülenkappe einen zylindrischen, die Kanüle umschließenden Vorsprung aufweist, der in der zweiten Rastposition des Verschlußteils dichtend der Innenmantelfläche der Nadelschutzkappe anliegt.

In verfahrensmäßiger Hinsicht wird die der Erfindung zugrundeliegende Aufgabe dadurch gelöst, dass nach dem Reinigen des aus Glas bestehenden Spritzenzylinders eine Einbrennsilikonisierung erfolgt, dass anschließend die Befüllung des Spritzenzylinders erfolgt, dass sodann die Kanülenkappe mit darin angeordneter Verschlußscheibe und in der ersten Rastposition aufgesetztem Verschlußteil auf den Spritzenzylinder aufgeprellt und danach die Spritze oder Karpule sterilisiert wird und schließlich das Verschlußteil in die zweite Rastposition niedergedrückt wird.

Hierbei besteht schließlich die vorteilhafte Möglichkeit, dass der Spritzenzylinder blasenfrei befüllt wird, so dass nach dem Aufsetzen der Kanülenkappe durch die plan dem stirnseitigen Ende des Spritzenzylinders anliegende Verschlußscheibe keine Lufteinschlüsse im Spritzenzylinder vorhanden sind.

Im folgenden wird die Erfindung an einem in der Zeichnung dargestellten Ausführungsbeispiel näher erläutert; es zeigen:
- Fig. 1: die Kanülenkappe mit der Verschlußscheibe sowie dem Verschlußteil in der Art einer Explosionsdarstellung,
- Fig. 2: den Gegenstand nach Fig. 1 in zum Aufsetzen auf die Spritze vorkonfektioniertem Zustand,
- Fig. 3: den Gegenstand nach Fig. 2, jedoch im auf die Spritze aufgesetzten Zustand,
- Fig. 4: den Gegenstand nach Fig. 3 im fertig konfektioniertem Zustand,
- Fig. 5: den Gegenstand nach Fig. 4, jedoch vorbereitet für die Applikation.

Die in der Zeichnung nur teilweise dargestellte Spritze oder Karpule ist vorgesehen für medizinische Zwecke und besteht aus einem Spritzenzylinder 1 aus Glas sowie einem in der Zeichnung nicht wiedergegebenen, im Spritzenzylinder 1 angeordneten Spritzenkolben, der mittels einer ebenfalls nicht dargestellten Kolbenstange verschiebbar ist. Diese Spritze ist dazu vorgesehen, im vorgefüllten Zustand und weitestgehend vorbereitet für eine unmittelbare Applikation in den Verkehr zu kommen.

Am kanülenseitigen Ende des Spritzenzylinders 1 ist axial eine Kanülenkappe 2 aufgesetzt, die dort im Rastsitz gehalten ist. In die Kanülenkappe 2 ist eine Kanüle 3 fest eingesetzt.

Ferner ist eine Verschlußscheibe 4 vorgesehen, die koaxial in der Kanülerikappe 2 angeordnet ist und einerseits stirnseitig dem Spritzenzylinder 1 und andererseits der innenseitigen Stirnfläche der Kanülenkappe 2 anliegt. Diese Verschlußscheibe 4 weist mittig eine Ausnehmung 5 in Form eines Sackloches auf, wobei der Boden dieser Ausnehmung 5 eine dünne Membran bildet.

Das dem Spritzenzylinder 1 zugewandte Ende der Kanüle 3 steht frei über die innenseitige Stirnfläche der Kanülenkappe 2 in die Ausnehmung 5 der Verschlußscheibe 4 hinein vor, so dass unter dem Druck des Spritzenkolbens, der über die Flüssigkeit auf die Membran übertragen wird, diese sich zur innenseitigen Spitze 6 der Kanüle 3 hin verformt und dadurch durchstochen bzw. zerstört wird. Damit ist die Spritze für der Anwendung selbstaktivierend ausgebildet.

Ferner ist ein die Kanüle 3 schützendes Verschlußteil 7 vorgesehen, dass aus einer Verschlußkappe 8 sowie einem Klemmring 9 besteht, wobei die Verschlußkappe 8 mit dem Klemmring 9 über eine Sollbruchstelle 10 verbunden ist. Diese Sollbruchstelle 10 ermöglicht es, die Verschlußkappe 8 vor der Anwendung der Spritze auf einfache Weise abzutrennen.

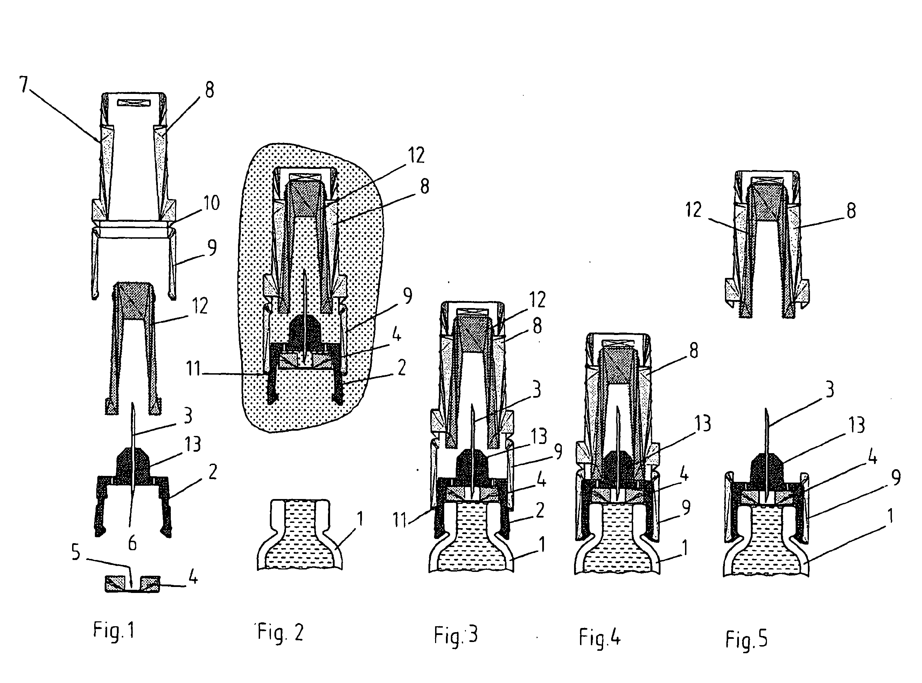

An der Außenmantelfläche der Kanülenkappe 2 ist für den Klemmring 9 eine erste Rastposition 11 vorgesehen, bei der eine Verbindung des Innenraums des Verschlußteils 7 mit der Umgebung besteht, so dass dieser Innenraum sterilisiert werden kann. Diese erste Rastposition 11 ergibt sich aus den Figuren 2 und 3. Nach erfolgter Sterilisierung kann das Verschlußteil 7 - noch unter sterilen Bedingungen - durch einfaches Aufdrücken in die in Figur 4 dargestellte zweite Rastposition verstellt werden, in der dann eine sterile Abdichtung gegenüber der Kanülenkappe 2 gegeben ist.

Zusätzlich ist im Inneren des Verschlußteils 7 eine aus Gummi bestehende Nadelschutzkappe 12 angeordnet.

Die Kanülenkappe 2 weist einen zylindrischen, die Kanüle 3 umschließenden Vorsprung 13 auf, der in der zweiten Rastposition des Verschlußteils 7 dichtend der Innenmantelfläche der Nadelschutzkappe 12 anliegt.

Durch diese Anordnung ist es möglich, auf einfach Weise eine Spritze bereitzustellen, zu deren Applikation lediglich die Verschlußkappe 8 abgetrennt werden muss. Insbesondere ist die Spritze bereits mit der Kanüle 3 versehen, ohne dass Einschränkungen im Prozessablauf in Kauf genommen werden müßten. Der Spritzenzylinder 1 kann also insbesondere Hochtemperaturprozessen, wie z. B. einer Einbrennsilikonisierung, unterworfen werden, ohne dass hierdurch der Sitz bzw. Halt der Kanüle beeinflusst wird. Darüber hinaus erlaubt die Anordnung eine blasenfreie Füllung des Spritzenzylinders 1 mit der pharmazeutischen Substanz.

## Patentansprüche

1. Vorgefüllte Spritze oder Karpule für medizinische Zwecke, mit einem Spritzenzylinder (1) aus Glas und einem darin angeordneten, mittels einer Kolbenstange verschiebbaren Spritzenkolben, ferner mit einer am kanülenseitigen Ende des Spritzenzylinders (1) axial aufgesetzten, im Rastsitz gehaltenen Kanülenkappe (2), in die eine Kanüle (3) fest eingesetzt ist, ferner mit einer zur Kanülenkappe (2) koaxial angeordneten, in die Kanülenkappe (2) eingesetzten Verschlußscheibe (4), die einerseits stirnseitig dem Spritzenzylinder (1) und andererseits der innenseitigen Stirnfläche der Kanülenkappe (2) anliegt und mittig eine Ausnehmung (5) in Form eines Sacklochs aufweist, deren Boden eine Membran bildet, wobei das dem Spritzenzylinder (1) zugewandte Ende der Kanüle (3) über die innenseitige Stirnfläche der Kanülenkappe (2) frei in die Ausnehmung (5) der Verschlußscheibe (4) vorsteht, sowie mit einem aus einer Verschlußkappe (8) und einem daran über eine Sollbruchstelle (10) angeschlossenen Klemmring (9) bestehenden Verschlußteil (7), das von einer ersten, an der Außenmantelfläche der Kanülenkappe (2) für den Klemmring (9) ausgebildeten Rastposition (11), die eine Sterilisierung des Verschlußteilinnenraums ermöglicht, in eine zweite, die Kanülenkappe (2) steril abdichtend umschließende Rastposition verstellbar ist.

2. Spritze oder Karpule nach Anspruch 1, **dadurch gekennzeichnet, daß** im Inneren des Verschlußteils (7) eine Nadelschutzkappe (12) angeordnet ist.

3. Spritze oder Karpule nach Anspruch 2, **dadurch gekennzeichnet, daß** die Nadelschutzkappe (12) aus Gummi besteht.

4. Spritze oder Karpule nach Anspruch 2 oder 3, **dadurch gekennzeichnet, daß** die Kanülenkappe (2) einen zylindrischen, die Kanüle (3) umschließenden Vorsprung (13) aufweist, der in der zweiten Rastposition des Verschlußteils (7) dichtend der Innenmantelfläche der Nadelschutzkappe (12) anliegt.

5. Verfahren zur Konfektionierung einer Spritze oder Karpule nach den Ansprüchen 1 bis 4, **dadurch gekennzeichnet, daß** nach dem Reinigen des aus Glas bestehenden Spritzenzylinders (1) eine Einbrennsilikonisierung erfolgt, daß anschließend die Befüllung des Spritzenzylinders (1) erfolgt, daß sodann die Kanülenkappe (2) mit darin angeordneter Verschlußscheibe (4) und in der ersten Rastposition (11) aufgesetztem Verschlußteil (7) auf den Spritzenzylinder (1) aufgeprellt und danach die Spritze oder Karpule sterilisiert wird und schließlich das Verschlußteil (7) in die zweite Rastposition niedergedrückt wird.

6. Verfahren nach Anspruch 5, **dadurch gekennzeichnet, daß** der Spritzenzylinder (1) blasenfrei befüllt wird.

## Claims

1. A pre-filled syringe or carpule for medical purposes comprising a syringe cylinder (1) of glass and a syringe piston which is arranged therein and which is displaceable by means of a piston stem, further comprising a cannula cap (2) which is fitted axially on the cannula end of the syringe cylinder (1) and which is held by a latching fit and into which a cannula (3) is fixedly inserted, further comprising a closure disc (4) which is arranged coaxially with respect to the cannula cap (2) and which is inserted into the cannula cap (2) and which on the one hand bears at the end against the syringe cylinder (1) and on the other hand against the inside end face of the cannula cap (2) and which centrally has a recess (5) in the form of a blind hole whose bottom forms a membrane, wherein the end of the cannula (3), which is towards the syringe cylinder (1), projects beyond the inside end face of the cannula cap (2) freely into the recess (5) of the closure disc (4), and comprising a closure portion (7) which comprises a closure cap (8) and a clamping ring (9) connected thereto by way of a desired-rupture location (10), the closure portion (7) being displaceable from a first latching position (11) which is provided at the outside peripheral surface of the cannula cap (2) for the clamping ring (9) and which permits sterilisation of the interior of the closure portion into a second latching position which encloses the cannula cap (2) in sterile sealing relationship.

2. A syringe or carpule according to claim 1 **characterised in that** a needle protection cap (12) is arranged in the interior of the closure portion (7).

3. A syringe or carpule according to claim 2 **characterised in that** the needle protection cap (12) comprises rubber.

4. A syringe or carpule according to claim 2 or claim 3 **characterised in that** the cannula cap (2) has a cylindrical projection (13) which surrounds the cannula (3) and which in the second latching position of the closure portion (7) bears sealingly against the inside peripheral surface of the needle protection cap (12).

5. A method of manufacturing a syringe or carpule according to claims 1 to 4 **characterised in that** after cleaning of the syringe cylinder (1) which comprises glass a stoving siliconisation operation is effected, that then the syringe cylinder (1) is filled, that then the cannula cap (2) with closure disc (4) arranged therein and with the closure portion (7) fitted in the first latching position (11) is pressed onto the syringe cylinder (1) and thereafter the syringe or carpule is sterilised and finally the closure portion (7) is pushed down into the second latching position.

6. A method according to claim 5 **characterised in that** the syringe cylinder (1) is filled bubble-free.

## Revendications

1. Seringue préremplie ou carpule à usage médical qui comporte:
- un cylindre de seringue (1) en verre dans lequel peut coulisser par l'intermédiaire d'une tige un piston de seringue,
- monté axialement sur l'extrémité du cylindre (1) situé du côté de la canule, un capuchon de canule (2) qui est maintenu dans le logement d'arrêt et dans lequel est insérée fixement une canule (3),
- coaxial au capuchon de canule et monté dans celui-ci, un disque de fermeture (4) est, d'un côté appliqué frontalement sur le cylindre de seringue (1) et de l'autre côté sur la face frontale interne du capuchon de canule (2) et il présente en son milieu un évidement (5) en forme de trou borgne dont le fond constitue une membrane, l'extrémité de la canule (3) située du côté du cylindre de seringue (1) faisant saillie sur la face frontale interne du capuchon de canule (2) en pénétrant librement dans l'évidement (5) du capuchon de canule (2),
- une partie de fermeture (7) est constituée d'un capuchon de fermeture (8) et d'une bague de serrage (9) qui lui est raccordée par l'intermédiaire d'une zone de rupture imposée (10), cette partie de fermeture pouvant passer d'une première position d'arrêt prévue pour la bague de serrage (9) sur la surface externe du capuchon de canule (2) et dans laquelle peut être stérilisé le volume interne de la partie de fermeture, à une seconde position d'arrêt dans laquelle elle enferme le capuchon de canule (2) de manière étanche et stérile.

2. Seringue ou carpule selon la revendication 1, **caractérisée en ce qu'**un capuchon de protection d'aiguille (12) est disposé à l'intérieur de la partie de fermeture (7).

3. Seringue ou carpule selon la revendication 2, **caractérisée en ce que** le capuchon de protection d'aiguille (12) est réalisé en caoutchouc.

4. Seringue ou carpule selon la revendication 2 ou 3, **caractérisée en ce que** le capuchon de canule (2) présente une saillie cylindrique (13) qui entoure la canule (3) et qui, dans la seconde position d'arrêt de la partie de fermeture est appliquée avec étanchéité sur la surface interne du capuchon de protection d'aiguille (12).

5. Procédé pour confectionner une seringue ou carpule selon les revendications 1 à 4, **caractérisé en ce qu'**après le nettoyage du cylindre de seringue (1) en verre, est effectuée une siliconisation au four suivie du remplissage du cylindre de seringue (1), puis le capuchon de canule (2) avec le disque de fermeture (4) qu'il comporte et la partie de fermeture (7) placée dans la première position d'arrêt (11), sont placés sur le cylindre de seringue (1), la seringue ou carpule est alors stérilisée et enfin la partie de fermeture (7) est rabattue à la deuxième position d'arrêt.

6. Procédé selon la revendication 5, **caractérisé en ce que** le cylindre de seringue (1) est rempli sans bulles.
